(19) Europäisches Patentamt
European Patent Office
Office européen des brevets

(11) Veröffentlichungsnummer : **0 368 225 B1**

(12) # EUROPÄISCHE PATENTSCHRIFT

(45) Veröffentlichungstag der Patentschrift :
**15.04.92 Patentblatt 92/16**

(51) Int. Cl.$^5$ : **C07C 239/20,** C07D 209/48

(21) Anmeldenummer : **89120555.1**

(22) Anmeldetag : **07.11.89**

(54) **Hydroxylaminderivate.**

Die Akte enthält technische Angaben, die nach dem Eingang der Anmeldung eingereicht wurden und die nicht in dieser Patentschrift enthalten sind.

(30) Priorität : **11.11.88 DE 3838310**

(43) Veröffentlichungstag der Anmeldung :
**16.05.90 Patentblatt 90/20**

(45) Bekanntmachung des Hinweises auf die Patenterteilung :
**15.04.92 Patentblatt 92/16**

(84) Benannte Vertragsstaaten :
**BE CH DE FR GB IT LI NL**

(56) Entgegenhaltungen :
**EP-A- 0 048 911**
**EP-A- 0 252 629**

(73) Patentinhaber : **BASF Aktiengesellschaft**
**Carl-Bosch-Strasse 38**
**W-6700 Ludwigshafen (DE)**

(72) Erfinder : **Wild, Jochen, Dr.**
**An der Marlach 7**
**W-6705 Deidesheim (DE)**
Erfinder : **Harreus, Albrecht, Dr.**
**Teichgasse 13**
**W-6700 Ludwigshafen (DE)**
Erfinder : **Schirmer, Ulrich, Dr.**
**Berghalde 79**
**W-6900 Heidelberg (DE)**
Erfinder : **Meyer, Norbert, Dr.**
**Dossenheimer Weg 22**
**W-6802 Ladenburg (DE)**
Erfinder : **Kast, Juergen, Dr.**
**Kastanienstrasse 24**
**W-6737 Boehl-Iggelheim (DE)**
Erfinder : **Kolassa, Dieter, Dr.**
**Moltkestrasse 8**
**W-6700 Ludwigshafen (DE)**

**EP 0 368 225 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Hydroxylaminderivate der allgemeinen Formel I

Aryl-A-O-B I

in der

Aryl einen ein- oder zweikernigen isocyclischen aromatischen Rest bedeutet, wobei dieser Rest folgende Substituenten tragen kann

- bis zu 5 Halogenatome und/oder
- bis zu 3 der nachstehenden Gruppen:

Nitro, Nitril, Hydroxy, Amino, $C_1$- bis $C_7$-Monoalkylamino, $C_2$- bis $C_{14}$-Dialkylamino, $C_1$- bis $C_7$-Alkyl, $C_1$- bis $C_7$-Alkoxy, $C_1$- bis $C_7$-Halogenalkoxy, $C_1$- bis $C_7$-Halogenalkyl, $C_1$- bis $C_7$-Alkylsulfido, $C_1$- bis $C_7$-Halogenalkylsulfido, $C_1$- bis $C_7$-Acyl, $C_1$- bis $C_7$-Alkoxycarbonyl, Carboxyl, $C_3$- bis $C_6$-Cycloalkyl und/oder Phenyl

in der

A eine 1,4-Butenylengruppe der allgemeinen Formeln IIa oder IIb ist,

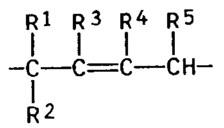

IIa

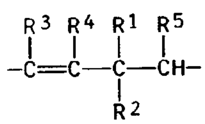

IIb

in der die Reste $R^1$ bis $R^4$ für Wasserstoff, Halogen, $C_1$-$C_6$-Alkyl und/oder $C_1$-$C_6$-Alkenyl stehen und $R^5$ Wasserstoff oder $C_1$-$C_6$-Alkyl bedeutet

und in der

B entweder für die $NH_2$-Gruppe oder für eine Dicarbonsäureimidgruppe der allgemeinen Formel III steht,

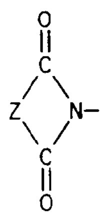

III

in der Z für einen Phenylen-, Naphthylen-, Pyridinylen-, Cyclopentylen-, Cyclohexylen-, Cyclohexenylen-, $C_2$- bis $C_4$-Alkenylen- oder $C_1$- bis $C_4$-Alkylenrest steht, wobei diese Reste Z unsubstituiert sind oder 1, 2, 3 oder 4 Halogen-, $C_1$- bis $C_4$-Alkyl- und/oder $C_1$- bis $C_4$-Halogenalkyl-Substituenten tragen können,

sowie, falls B eine Aminogruppe bedeutet, Salze der Verbindungen I.

Weiterhin betrifft die vorliegende Erfindung Verfahren zur Herstellung der substituierten Hydroxylaminderivate der allgemeinen Formel I.

In der EP-B 48 911 sind substituierte Hydroxylaminderivate beschrieben, welche als Ausgangsstoffe zur Herstellung von Antidiabetica dienen. Zu der Vielzahl der in dieser Patentschrift beanspruchten Verbindungen zählen formal auch solche der allgemeinen Formel I, jedoch enthält die Beschreibung weder einen Hinweis wie und aus welchen Ausgangsmaterialien diese Verbindungen hergestellt werden können, noch sind darin Daten zur physikalischen Charakterisierung solcher Verbindungen aufgeführt. Die Beispiele dieser Patentschrift behandeln lediglich die Herstellung solcher Hydroxylamine, in denen der Rest A eine Propenylen-, Propoxylen- oder Butylengruppe ist.

Die Erfindung lag die Aufgabe zugrunde, als Vorprodukte geeignete Verbindungen für die Herstellung von Herbiziden der allgemeinen Formel VIII

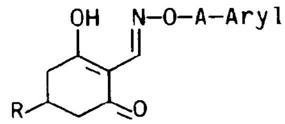

VIII

sowie Verfahren zur Herstellung dieser Vorprodukte bereitzustellen.

Dementsprechend wurden die eingangs definierten, substituierten Hydroxylaminderivate der allgemeinen Formel I gefunden.

Weiterhin wurde ein Verfahren zur Herstellung der substituierten Hydroxylaminderivate der allgemeinen Formel I, gefunden, das dadurch gekennzeichnet ist, daß man Verbindungen der allgemeinen Formel IV

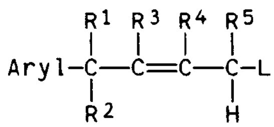

IV

oder V,

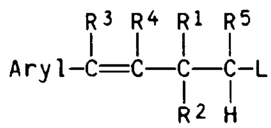

V

in denen L eine unter nucleophilen Bedingungen substituierbare Abgangsgruppe ist, zunächst mit einem Hydroxyimid der allgemeinen Formel VI

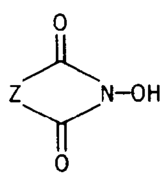

VI

in Gegenwart eines Lösungsmittels und einer Base bei Temperaturen von 0 bis 140°C umsetzt und aus den erhaltenen Imidethern die Hydroxylaminderivate I, in denen B für die $NH_2$-Gruppe steht, mittels Basen oder Säuren freisetzt.

Ferner wurde eine Ausgestaltung des Verfahrens zur Herstellung der substituierten Hydroxylaminderivate der allgemeinen Formel Ia,

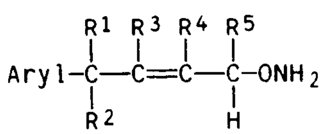

Ia

gefunden, die dadurch gekennzeichnet ist, daß man eine Verbindung der allgemeinen Formel VII,

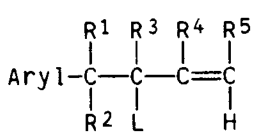

VII

worin L eine unter nucleophilen Bedingungen substituierbare Abgangsgruppe ist oder Mischungen der Verbindung der allgemeinen Formel VII mit der isomeren Verbindung der allgemeinen Formel IV

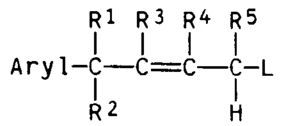 IV

zunächst mit einem Hydroxyimid der allgemeinen Formel VI

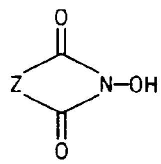 VI

in Gegenwart eines Lösungsmittels und einer Base bei Temperaturen von 0 bis 140°C umsetzt und aus den erhaltenen Imidethern die Hydroxylaminderivate Ia mittels Basen oder Säuren freisetzt.

In den erfindungsgemäßen Hydroxylaminderivaten der allgemeinen Formel I ist die Gruppe Aryl- ein ein- oder zweikerniger, isocyclischer, aromatischer Rest, bevorzugt die Phenyl- oder Naphthylgruppe.

Die Arylgruppen können unsubstituiert vorliegen oder auch mit gleichen oder verschiedenen Substituenten substituiert sein. Im allgemeinen tragen die isocyclischen Arylgruppen 1 bis 5 Halogenatome und/oder 1 bis 3 Nitro-, Cyano-, Hydroxy-, Amino-, $C_1$- bis $C_7$-Monoalkylamino-, $C_2$- bis $C_{14}$-Dialkylamino-, $C_1$- bis $C_7$- Alkyl-, $C_1$- bis $C_7$-Alkoxy-, $C_1$- bis $C_7$-Halogenalkyl-, $C_1$- bis $C_7$-Halogenalkoxy-, $C_1$- bis $C_7$-Alkylsulfido-, $C_1$- bis $C_7$-Halogenalkylsulfido-, $C_1$- bis $C_7$-Alkoxycarbonyl-, Carboxyl-, $C_1$- bis $C_7$-Acyl-, $C_3$- bis $C_6$-Cycloalkyl- und/oder Phenylgruppen.

Als Halogenatome können sowohl Fluor-, Chlor-, Brom- als auch Jodatome an die Arylgruppen gebunden sein, bevorzugt ist jedoch deren Substitution mit Fluor, Chlor und/oder Brom.

Von den $C_1$- bis $C_7$-Monoalkylamino- und $C_2$- bis $C_{14}$-Dialkylamino-Substituenten sind die $C_1$- bis $C_4$-Monoalkylamino- und die $C_2$- bis $C_8$-Dialkylamino-Substituenten bevorzugt. Dabei können die ans Stickstoffatom gebundenen Alkylgruppen sowohl geradkettig oder verzweigt sein. Cycloalkylgruppen können gleichfalls ans Stickstoffatom gebunden sein. sein. Bevorzugt seien die Monomethylamino-, Dimethylamino-, Ethylamino-, Diethylamino-, Propylamino-, Butylamino-, tert.-Butylamino-, iso-Butylamino-, Hexylamino-, Cyclohexylamino-, Dibutylamino-, Methyl-hexylamino-, Methyl-ethylamino-, Methyl-cyclohexylamino-, Cyclopropylamino-, Cyclopentylamino und die Heptylamino-Gruppe genannt.

Unter Dialkylaminogruppen sind auch solche Gruppen zu verstehen, in denen die ans Stickstoffatom gebundenen Alkylreste zu einem Ring verbunden sind, beispielsweise die Aziridinyl-, Pyrrolidinyl- und Piperidinyl-Gruppe.

Die Alkylsubstituenten des Arylsystems können geradkettig, verzweigt oder cyclisiert sein. Bevorzugte Alkylsubstituenten sind $C_1$- bis $C_4$-Alkylgruppen, insbesondere die Methyl-, Ethyl-, Butyl-, Isopropyl- und tert.Butylgruppe sowie die Cyclopropyl-, Cyclopentyl- und die Cyclohexylgruppe.

Von den $C_1$- bis $C_7$-Alkoxy- und $C_1$- bis $C_7$-Alkylsulfidosubstituenten sind diejenigen mit 1 bis 4 Kohlenstoffatomen bevorzugt. Besonders geeignete Substituenten sind die Methoxy-, Ethoxy-, Propoxy-, Butoxy-, Isopropoxy-, tert.-Butoxy-, Methylsulfido- und die Ethylsulfido-Gruppe.

Die $C_1$- bis $C_7$-Halogenalkyl-, $C_1$- bis $C_7$-Halogenalkoxy- und $C_1$- bis $C_7$-Halogenalkylsulfido-Substituenten können perhalogeniert sein oder auch noch Wasserstoffatome enthalten. Sind diese Substituenten mit nur einer Halogenspezies perhalogeniert, so sind die bevorzugten Halogene das Fluor und das Chlor, bei Perhalogenierung mit verschiedenerlei Halogenatomen können diese Gruppen neben Fluor und Chlor auch noch Brom enthalten. Jodatome finden sich im allgemeinen nur in solchen halogenierten Substituenten, welche zusätzlich noch Wasserstoff enthalten. Es versteht sich von selbst, daß nur solche halogenierte Substituenten in Betracht gezogen werden, welche sich unter den Bedingungen der Verfahren zur Herstellung der erfindungsgemäßen Hydroxylaminderivate inert verhalten. Bevorzugt werden solche Substituenten mit 1 bis 4 Kohlenstoffatomen. Vorzugsweise verwendete Substituenten sind: $CF_3$, $CF_2Cl$, $CFCl_2$, $CF_3$-$CF_2$, $C_4F_9$, $CF_2Br$, $CF_3$-$CH_2$, $HCF_2$-$CF_2$, $HCFCl$-$CF_2$, $CCl_3$, $CF_3$-O, $F_2CCl$-O, $CCl_3$-O, $CF_3$-$CH_2$-O, $HCF_2$-$CF_2$-O, $F_3C$-S.

Die Arylgruppen der erfindungsgemäßen Hydroxylaminderivate können weiterhin als Substituenten 1 bis 3 Carboxylgruppen tragen, welche mit $C_1$- bis $C_7$-Alkoholen verestert sein können. Bevorzugt sind solche Alkoxycarbonylgruppen, deren Alkoxykomponente 1 bis 4 Kohlenstoffatome hat.

Geeignete Substituenten sind außerdem $C_1$- bis $C_7$-Acylgruppen, insbesondere $C_1$- bis $C_4$-Acylgruppen.

Besonders bevorzugte Gruppen Aryl-, sind z.B. die Phenyl-, Naphthyl-, 4-Chlorphenyl-, 3-Chlorphenyl-, 2-Chlorphenyl-, 4-Fluorphenyl-, 3-Fluorphenyl-, 2-Fluorphenyl-, 4-Bromphenyl-, 3-Bromphenyl-, 2-Bromphenyl-, 2,4-Difluorphenyl-, 2,4-Dichlorphenyl-, 2,4-Dibromphenyl-, 3,4-Difluorphenyl-, 3,4-Dichlorphenyl-, 3,4-Dibromphenyl-, 2,3-Difluorphenyl, 2,3-Dichlorphenyl-, 2,3-Dibromphenyl-, 2,5-Difluorphenyl-, 2,5-Dichlorphenyl-, 2,5-Dibromphenyl-, 2,6-Difluorphenyl-, 2,6-Dichlorphenyl-, 2,6-Dibromphenyl, 3,5-Difluorphenyl-, 3,5-Dichlorphenyl-, 3,6-Difluorphenyl-, 3,6-Dibromphenyl-, 3,6-Dichlorphenyl-, 2,4,6-Trichlorphenyl-, 2-Chlor-4-fluorphenyl-, 2-Brom-4-fluorphenyl-, 3-Chlor-4-fluorphenyl-, 2-Methylphenyl-, 3-Methylphenyl-, 4-Methylphenyl-, 2,3-Dimethylphenyl-, 2,4-Dimethylphenyl-, 2,6-Dimethylphenyl-, 2,4,6-Trimethylphenyl-, 4-tert-butylphenyl-, 2,6-Dimethyl-4-tert-butylphenyl-, 2-Chlor-4-methylphenyl-, 2-Nitrophenyl-, 3-Nitrophenyl-, 4-Nitrophenyl-, 2,4-Dinitrophenyl-, 2-Fluor-4-nitrophenyl-, 2-Chlor-4-nitrophenyl-, 2-Nitro-4-fluorphenyl-, 2-Nitro-4-chlorphenyl-, 2-Hydroxy-3-nitrophenyl-, 2-Hydroxy-4-nitrophenyl-, 3-Hydroxy-4-nitrophenyl-, 2-Hydroxyphenyl-, 3-Hydroxyphenyl-, 4-Hydroxyphenyl-, 2,4-Dihydroxyphenyl-, 2,5-Dihydroxyphenyl-, 2,6-Dihydroxyphenyl-, 3,5-Dihydroxyphenyl-, 3-Methyl-4-hydroxyphenyl-, 2-Methyl-4-hydroxyphenyl-, 2-Methoxyphenyl-, 4-Methoxyphenyl-, 3-Methoxy-4-hydroxyphenyl-, 3,4-Dimethoxyphenyl-, 2-Carboxyphenyl-, 2-Acetylphenyl, 4-Acetylphenyl, 2-Methoxycarbonylphenyl-, 4-Methoxycarbonylphenyl-, 4-Butoxycarbonylphenyl-, 2-Amino-3-acetylphenyl-, 3-Methylaminophenyl-, 4-Methylaminophenyl-, 3-N,N-Dimethylaminophenyl-, 3-Cyclopropylaminophenyl-, 4-Trifluormethylphenyl-, 4-Trichlormethylphenyl-, 4-Monochlormethylphenyl-, 4-Dichlorfluormethylphenyl-, 4-Difluormethylphenyl-, 2-Nitrilophenyl-, 3-Nitrilophenyl- und die 4-Nitrilophenyl-Gruppe.

Der Rest A der erfindungsgemäßen Hydroxylaminderivate ist eine Butenylengruppe der allgemeinen Formeln IIa

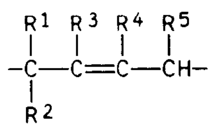

IIa

oder IIb.

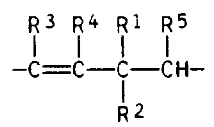

IIb

Dementsprechend steht die Doppelbindung der Butenylengruppe entweder in Konjugation mit dem aromatischen System oder ist von diesem durch eine $CR^1R^2$-Gruppe getrennt. $R^1$, $R^2$, $R^3$, $R^4$ und $R^5$ stehen in den beiden Formeln für Wasserstoffatome und/oder die Substituenten Halogen, $C_1$- bis $C_6$-Alkyl- und/oder $C_1$- bis $C_6$-Alkenyl. Somit kann der Rest A unsubstituiert sein oder 1 bis 4 gleiche oder verschiedene der genannten Substituenten tragen.

Bevorzugte Reste A sind solche, in denen
$R^1$, $R^2$ und $R^5$ für Wasserstoffatome und/oder $C_1$- bis $C_4$-Alkylgruppen und $R^3$ und $R^4$ für Wasserstoffatome, Halogenatome und/oder $C_1$- bis $C_4$-Alkylgruppen stehen.

Bevorzugte Halogensubstituenten des Rests A sind Fluor und Chlor, ein besonders bevorzugter Alkylsubstituent ist die Methylgruppe.

Der Rest B der erfindungsgemäßen Hydroxylaminderivate ist entweder eine Aminogruppe oder steht für eine Dicarbonsäureimidgruppe der allgemeinen Formel III,

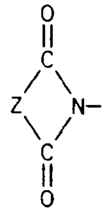

III

in der Z für einen Phenylen-, Naphthylen, Pyridinylen-, Cyclopentylen-, Cyclohexylen-, Cyclohexenylen-, $C_2$- bis $C_4$-Alkenylen- oder $C_2$- bis $C_4$-Alkylenrest bedeutet, der unsubstituiert sein kann oder gegebenenfalls 1, 2,

3 oder 4 der Substituenten Halogen, $C_1$- bis $C_4$-Alkyl- und/oder $C_1$- bis $C_4$-Halogenalkyl tragen kann.

Steht die Gruppe Z für einen cyclischen, aromatischen oder heteroaromatischen Rest, so versteht es sich von selbst, daß es sich bei III um Dicarbonsäureimidgruppe solcher Dicarbonsäuren handelt, in denen die Carboxylgruppen in 1,2-Stellung zueinander stehen. Unter Naphthylen- sind somit die Reste

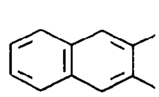 , 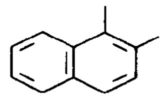 ,

unter Pyridinylen- die Reste

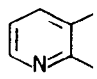 , 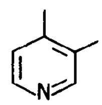

und unter Cyclohexenylen- die Reste

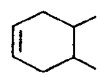 , 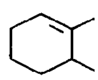 , 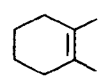

zu verstehen.

Sind die Reste Z substituiert, so kann das Substitutionsmuster beliebig sein. Bevorzugt sind jedoch unsubstituierte Reste Z. Besonders bevorzugt aufgrund der leichten und kostengünstigen Verfügbarkeit der Ausgangsmaterialien sind die Hydroxylaminderivate, in denen Z eine $C_2$- bis $C_3$-Alkylen, $C_2$- bis $C_4$-Alkenylen- und insbesondere eine Phenylengruppe ist.

Die erfindungsgemäßen Hydroxylaminderivate in denen der Rest B für eine Dicarbonsäureimidgruppe steht, sind unmittelbare Vorprodukte zur Herstellung der erfindungsgemäßen Hydroxylaminderivate, worin B eine Aminogruppe ist.

Sowohl die Hydroxylaminderivate in denen B eine Dicarbonsäureimidgruppe darstellt als auch die eine freie Aminogruppe enthaltenden Hydroxylaminderivate sind stabile Verbindungen und können als solche isoliert, gelagert und weiterverarbeitet werden. Zur Isolierung der Hydroxylaminderivate mit freier Aminogruppe kann es sich jedoch als vorteilhaft erweisen, wenn diese in ihre Salze mit organischen oder anorganischen Säuren überführt werden, da diese Salze leichter kristallin zu erhalten sind. Darüberhinaus kann durch die Wahl des Säureanions das Löslichkeitsverhalten dieser Hydroxylammoniumsalze in organischen Lösungsmitteln oder Wasser gezielt beeinflußt werden - eine Maßnahme, welche die Weiterverarbeitung der erfindungsgemäßen Hydroxylaminderivate erleichtert. Besonders bevorzugt werden die aus den entsprechenden Hydroxylaminderivaten hergestellten Hydroxylammoniumsalze mit Anionen wie Chlorid, Bromid, Sulfat, Nitrat, Phosphat, Formiat, Acetat, Malonat, Oxalat, Methansulfonat, Benzolsulfonat und Toluolsulfonat genutzt.

Zur Herstellung der erfindungsgemäßen Hydroxylaminderivate Ia

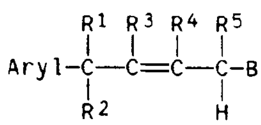

Ia

und Ib

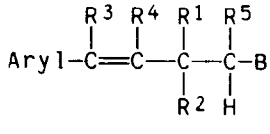

Ib

geht man von Verbindungen der allgemeinen Formeln IV

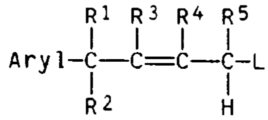

IV

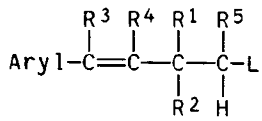

V

und VII

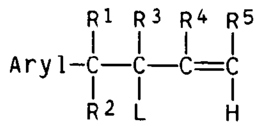

VII

aus, in denen L für eine unter nucleophilen Bedingungen substituierbare Abgangsgruppe steht. Bevorzugte Abgangsgruppen L sind die Halogenide Chlorid, Bromid und Jodid und die Sulfonsäureester der Methansulfonsäure, Trifluormethansulfonsäure, Benzolsulfonsäure, Brombenzolsulfonsäure oder der Toluolsulfonsäure. Besonders bevorzugte Abgangsgruppen sind die Halogenide Chlorid und Bromid sowie Methansulfonat und Toluolsulfonat.

Zur Darstellung der erfindungsgemäßen Hydroxylaminderivate in denen B eine Dicarbonsäureimidgruppe ist, werden die Ausgangsverbindungen der allgemeinen Formel IV, V und/oder VII mit Dicarbonsäurehydroxyimiden der allgemeinen Formel VI

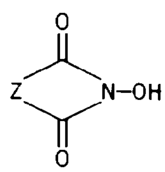

VI

umsetzt. Aus den erhaltenen Imidethern werden anschließend die erfindungsgemäßen Hydroxylaminderivate, in denen B eine Aminogruppe ist, durch Abspaltung der entsprechenden Dicarbonsäurereste erhalten.

Befindet sich die Abgangsgruppe L wie in den Ausgangsverbindungen der allgemeinen Formeln IV und V in ω-Stellung zum Arylsystem so ist bei deren Substitution durch die Hydroxyimide VI nicht mit einer Isomerisierung der Doppelbindung zu rechnen, und man erhält die entsprechenden Hydroxylaminderivate Ia und Ib, in denen B eine Dicarbonsäureimidgruppe ist. Werden zu dieser Umsetzung hingegen die isomeren Ausgangsverbindungen der allgemeinen Formeln VII verwendet, so findet im Zuge der Umsetzung im allgemeinen eine praktisch quantitative Isomerisierung der Doppelbindung statt, und es entstehen die Hydroxylaminderivate der allgemeinen Formel Ia.

Dieser überraschende Effekt ist insofern günstig, da er es ermöglicht, aus Mischungen der isomeren Ausgangsverbindungen IV und VII, wie sie zwangsweise bei der Herstellung der Ausgangsverbindungen IV durch Meerwein-Arylierung der entsprechenden Arylverbindungen mit Butadienen erhalten werden, zu einem einheitlichen Produkt Ia zu gelangen. Es erübrigt sich somit eine aufwendige Trennung solcher Isomerengemische.

Die Umsetzung der Ausgangsverbindungen IV, V und/oder VII mit den Hydroxyimiden wird zweckmäßigerweise in Gegenwart einer Base ausgeführt. Geeignet sind prinzipiell alle Basen, die in der Lage sind, die Hydroxyimide VI zu deprotonieren ohne das Imidsystem anzugreifen. Dies sind insbesondere die sogenannten nicht-nucleophilen Basen. Beispielsweise genannt seien Mineralbasen wie Alkalimetall- und Erdalkalimetallcarbonate, Alkalimetall- und Erdalkalimetallhydrogencarbnate, organische Basen wie aliphatische, cycloaliphatische und aromatische tertiäre Amine. Es können auch Gemische dieser Basen verwendet werden.

Als Einzelverbindungen seien folgende Basen beispielhaft aufgeführt: Natriumcarbonat, Kaliumcarbonat, Magnesiumcarbonat, Calciumcarbonat, Bariumcarbonat, die Hydrogencarbonate dieser Metalle, Trimethylamin, Triethylamin, Tributylamin, Ethyldiisopropylamin, N,N-Dimethylanilin, 4-N,N-Dimethylaminopyridin, Diazobicyclooctan, Diazobicycloundecan, N-Methylpiperidin, 1,4-Dimethylpiperazin, Pyridin, Chinolin, Bipyridin, Phenanthrolin. Bevorzugt sind die preiswerten Basen Natrium- und Kaliumcarbonat.

Die Base wird im allgemeinen in äquivalenten Mengen bis zu einem Überschuß von 5 Äquivalenten, bezogen auf das Hydroxyimid, zugegeben. Ein höherer Überschuß ist möglich, entbehrt aber zusätzliche Vorteile. Die Verwendung einer geringen Basenmenge ist ebenfalls möglich. Bevorzugt wird jedoch eine Basenmenge von 1 bis 3, insbesondere von 1 bis 2 Äquivalenten, bezogen auf das Hydroxyimid VI eingesetzt.

Die Verwendung von nucleophilen Basen, z.B. Alkalimetall- und Erdalkalimetallhydroxiden, insbesondere Natrium- und Kaliumhydroxid, ist ebenfalls möglich. In diesem Falle ist es vorteilhaft, die Base in äquivalenten Mengen bezüglich des Hydroxyimids VI einzusetzen, um einem nucleophilen Angriff der Hydroxylionen auf die Carbonylfunktion der Imidgruppierung vorzubeugen.

Zweckmäßigerweise setzt man die Ausgangsverbindungen IV, V und/oder VII mit den Hydroxyimiden VI in einem Lösungsmittel um, das sich unter den Reaktionsbedingungen inert verhält. Vorteilhafte Lösungsmittel sind z.B. polare-aprotische Lösungsmittel wie Dimethylformamid, N-Methylpyrrolidon, Dimethylsulfoxid, Sulfolan und cyclische Harnstoffe. Die Lösungsmittelmenge ist im allgemeinen nicht kritisch.

Die Umsetzung der Ausgangsverbindungen IV, V und/oder VII mit den Hydroxyimiden VI kann auch unter Anwendung der Phasentransfer-Katalyse ausgeführt werden. In diesem Falle werden mit Wasser zwei Phasen bildende Lösungsmittel, bevorzugt Chlorkohlenwasserstoffe, eingesetzt. Als Phasentransferkatalysatoren eignen sich die üblicherweise zu solchen Zwecken verwendeten quartären Ammonium- und Phosphoniumsalze, Polyethylenglykole, Polyethylenglykolether und Kronenether, wie sie z.B. in Dehmlow et al., Phase Transfer Catalysis, S. 37 - 45 und S. 86 - 93, Verlag Chemie, Weinheim 1980, beschrieben sind. Die Phasentransferkatalysatoren werden zweckmäßigerweise in Mengen von 1 bis 10 Vol%, bevorzugt in Mengen von 3 bis 5 Vol%, bezogen auf das Volumen der Reaktionsmischung, eingesetzt.

Die Umsetzung der Ausgangsverbindungen IV, V und/oder VII mit den Hydroxyimiden VI wird im allgemeinen im Temperaturbereich zwischen 0 und 140°C, bevorzugt zwischen 20 und 100°C, insbesondere zwischen 40 und 80°C, durchgeführt. Zweckmäßigerweise wird dabei so vorgegangen, daß man das Hydroxyimid VI zusammen mit der Base im Lösungsmittel vorlegt und das Ausgangsmaterial IV, V und/oder VII zu dieser Lösung dosiert. Dabei kann es sich als günstig erweisen, wenn das Hydroxyimid bei einer tieferen Temperatur, bespielsweise bei 0 bis 50°C, zugegeben und die Reaktionsmischung erst nach dieser Zugabe auf die eigentliche Reaktionstemperatur erhitzt wird.

Nach beendeter Reaktion wird die abgekühlte Reaktionsmischung zweckmäßigerweise mit Wasser versetzt, wobei sich die gebildeten Hydroxylaminderivate I, in denen B eine Dicarbonsäureimidgruppe ist, als kristalline Festkörper oder als Öle abscheiden. Die auf diese Weise erhaltenen Hydroxylaminderivate I können, falls gewünscht, durch Umkristallisation oder durch Extraktion weiter gereinigt werden.

Die Hydroxylaminderivate I, in denen B eine Dicarbonsäureimidgruppe ist können zwischengelagert werden oder sogleich in die Hydroxylaminderivate, in denen B eine freie Aminogruppe ist, umgewandelt werden. Diese Umwandlung kann nach an sich bekannten Verfahren durchgeführt werden, wie sie beispielsweise in DE-A 3 615 973 und den darin zitierten Schriften beschrieben sind. Bevorzugt wird das Verfahren gemäß DE-A 3 615 973 angewandt, nach dem die Hydroxylaminderivate I mit freier Aminogruppe B mittels Ethanolamin freigesetzt werden. Die Freisetzung der Hydroxylaminderivate I mit freier Aminogruppe B mit Hilfe anderer Basen wie wäßrigen Mineralbasen, mit Aminen, Hydrazinen, Hydroxylaminen oder mittels wäßriger Säuren ist ebenfalls möglich.

Aus den nach diesen Verfahren erhaltenen Reaktionsgemischen können die Hydroxylaminderivate I mit freier Aminogruppe B mittels üblicher Aufarbeitungsmethoden isoliert werden, beispielsweise durch Extraktion oder durch Kristallisation. Zur Erhöhung der Kristallisationstendenz dieser Hydroxylaminderivate kann es oftmals förderlich sein, diese in ihre Salze mit Mineralsäuren oder organischen Säuren überzuführen. Dazu werden im allgemeinen verdünnte Lösungen dieser Säuren mit den Hydroxylaminderivaten umgesetzt, und zwar zweckmäßigerweise in äquivalenten Mengen. Die erhaltenen Hydroxylammoniumsalze können wie die Hydroxylaminderivate mit freier Aminogruppe direkt zu den Herbiziden der Formel VIII weiterverarbeitet werden oder auch, falls gewünscht, gelagert werden.

Die Ausgangsverbindungen der allgemeinen Formeln IV, V und VII sind bekannt oder nach bekannten Verfahren herstellbar.

Beispielsweise können die Verbindungen IV und VII durch Meerwein-Arylierung der entsprechenden aromatischen Diazoniumsalze mit Butadien oder entsprechend substituierten Butadienen erhalten werden, wobei als Produkt ein Gemisch der isomeren Verbindungen IV und VII im Verhältnis von 70-80:20-30 anfällt. Weitere Erläuterungen zu diesem Herstellverfahren werden in Organic Reactions 11, 189-260 (1960) und Organic Reactions 24, 225-259 (1976) gegeben. Die Doppelbindung der nach diesem Verfahren hergestellten Verbindungen IV und VII hat die trans-Konfiguration.

Die Verbindungen der allgemeinen Formel IV mit cis-Konfiguration der Doppelbindung können nach dem Verfahren von Dupont et al (Bull. Soc. Chem. Fr. 653, (1954)) oder von Morgan et al (J. Med. Chem. 29, 1398 (1986)) erhalten werden.

Die Ausgangsverbindungen V mit trans-Konfiguration der Doppelbindung sind beispielsweise durch Grignard-Reaktion der betreffenden Arylhalogenide mit unsubstituierten oder entsprechend den Substituenten $R^1$, $R^2$, $R^3$, $R^4$ und $R^5$ substituierten Cyclopropanalen oder Cyclopropylalkyl-ketonen und anschließende Dehydratisierung der erhaltenen Cyclopropancarbinole - mit einhergehender Öffnung des Cyclopropanringes - zugänglich.

Die Grignard-Reaktion kann dabei nach Standardmethoden durchgeführt werden. Als Dehydratisierungsreagenzien eignen sich die üblichen Dehydratisierungsmittel wie Brönstedt-Säuren und Lewis-Säuren. Die Wahl des im Einzelfalle angewandten Dehydratisierungsmittels wird im allgemeinen von der gewünschten Abgangsgruppe L bestimmt, welche im Zuge der Dehydratisierungsreaktion in das Molekül eingeführt wird. Beispielhaft seien als geeignete Dehydratisierungsmittel Salzsäure, Bromwasserstoffsäure, Jodwasserstoffsäure, Toluolsulfonsäure, Methansulfonsäure, Benzolsulfonsäure, Brombenzolsulfonsäure, Zinkchlorid, Zinkbromid, Zinkjodid, Magnesiumbromid, Magnesiumjodid, Phosphortrichlorid, Phosphorpentachlorid, Phosphoroxychlorid, Phosphortribromid und Phosphortrijodid genannt. Die Anwendung der genannten Brönstedt-Säuren wird im allgemeinen bevorzugt. Durch die Wahl des Dehydratisierungsmittels hat man es außerdem in der Hand, gegebenenfalls weitere Substituenten, insbesondere die Substituenten Chlorid, Bromid und Jodid in die Butenylen-Gruppe einzuführen. Hierzu sind im allgemeinen die genannten Phosphorhalogenide besonders geeignet. Im übrigen ist die Dehydratisierung von Cyclopropancarbinolen zu den entsprechenden Alkenen bekannt und Beispiele zur Durchführung dieser Reaktion können z.B. den Literaturstellen J. Org. Chem. 45, 2566 (1980); J. Am. Chem. Soc. 90, 2882 (1968); Bull. Soc. Chim. Fr. 1849 (1961) und J. Org. Chem. 37, 922 (1972) entnommen werden.

Zur Darstellung der Ausgangsverbindungen V mit cis-Konfiguration der Doppelbindung kann ebenfalls auf bekannte Verfahren, wie sie beispielsweise in J. Med. Chem. 24, 678 (1981) und J. Chem. Ecol. 10, 1201 (1984) beschrieben sind, zurückgegriffen werden.

Die erfindungsgemäßen Verbindungen sind Vorprodukte für Herbizide der allgemeinen Formel VIII.

Beispiele

Beispiele 1 bis 7

Beispiel 1

E-N-(4-Phenyl-2-butenyloxy)phthalimid (1)

78,3 g (0,48 mol) N-Hydroxyphthalimid und 44,2 g (0,32 mol) Kaliumcarbonat wurden in 480 ml trockenem N-Methylpyrrolidon vorgelegt. Zu dieser Mischung wurden bei einer Temperatur von 40°C 88,8 g (0,54 mol) einer Mischung der isomeren Arylbutenylchloride IX und X, in der

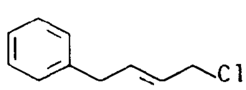

IX

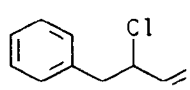

X

diese im Isomerenverhältnis von 78:22 vorlagen, getropft. Nach der Zugabe wurde noch 6 h auf 60°C erwärmt. Anschließend wurde die abgekühlte Reaktionsmischung auf Eiswasser gegossen, wobei sich das Produkt kristallin abschied. Das Produkt wurde unter Anwendung von Unterdruck filtriert, gewaschen und getrocknet.

Auf analoge Weise wurden die Verbindungen (2), (3), (4), (5), (6) und (7) hergestellt. Die Strukturformeln, Ausbeuten und physikalischen Daten dieser Verbindungen sind in Tabelle 1 aufgelistet.

Auf analoge Weise können die in Tabelle 2 aufgeführten Verbindungen (8) bis (13) hergestellt werden.

Tabelle 1

| Verbindung | Ausbeute | Schmelzpunkt [°C] | $^1$H-NMR-Daten: δ (ppm) |
|---|---|---|---|
| 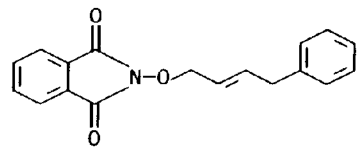 (1) | 90 % | 70 - 71 | $^1$H-NMR (DMSO-$d_6$): δ (ppm):<br>3,35 (d, 2H, Ar-$CH_2$);<br>4,62 (d, 2H, $CH_2$-O);<br>5,4-5,85 (m, 1H, Ar-$CH_2$-CH=);<br>5,85-6,05 (m, 1H, =CH-$CH_2$-O);<br>7,0-7,2 (m, 5H, Ar-H);<br>7,85 (s, 4H, Phthalimid-Protonen)<br>DMSO-$d_6$: perdeuteriertes Dimethylsulfoxid |
| 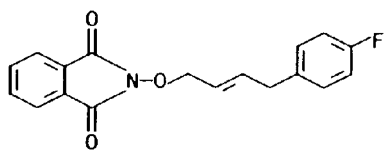 (2) | 85 % | 60 - 62 | in $CDCl_3$: 3,3 (d, 2H, Ar-$CH_2$);<br>4,68 (d, 2H, -$CH_2$-O-);<br>5,7-6,0 (m, 2H, -CH=CH-);<br>6,7-7,1 (2d, 4H, Ar-H);<br>7,7-7,9 (m, 4H, Phthalimid-Protonen) |
| 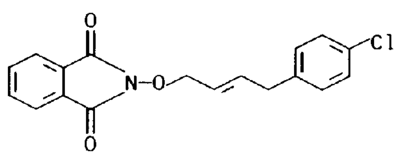 (3) | 83 % | 88 - 91 | in DMSO-$d_6$: 3,3 (d, 2H, Ar-$CH_2$);<br>4,6 (d, 2H, -$CH_2$-O-);<br>5,6-6,0 (m, 2H, -CH=CH-);<br>7,0-7,3 (2d, 4H, Ar-H);<br>7,84 (s, 4H, Phthalimid-Protonen) |

Tabelle 1 (Fortsetzung)

| Verbindung | Ausbeute | Schmelzpunkt [°C] | 1H-NMR-Daten: δ (ppm) |
|---|---|---|---|
| 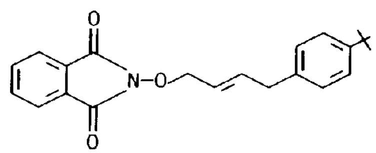 (4) | 85 % | Öl | in $CDCl_3$: 1,2 (s, 9H, tert.-Butyl); 3,35 (d, 2H, Ar-$CH_2$-); 4,66 (d, 2H, $CH_2$-O); 5,7-6,0 (m, 2H, -CH=CH-); 6,9-7,2 (2d, 4H, Ar-H); 7,7-7,9 (m, 4H, Phthalimid-Protonen) |
| 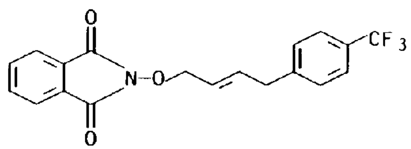 (5) | 91 % | 88 - 89 | in $CDCl_3$: 3,4 (d, 2H, Ar-$CH_2$); 4,7 (d, 2H, -$CH_2$-O-); 5,7-6,0 (m, 2H, -CH=CH-); 7,15-7,2 (2d, 4H, Ar-H); 7,7-7,9 (m, 4H, Phthalimid-Protonen) |
| 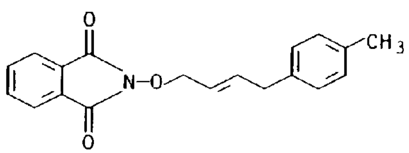 (6) | 33 | 86 - 87 | in DMSO-$d_6$: 2,24 (s, 3H, -$CH_3$) 3,28 (d, 2H, Ar-$CH_2$); 4,65 (d, 2H, -$CH_2$-O); 5,6-6,1 (2m, 2H, -CH=CH-); 6,95 (s, 4H, Ar-H); 7,8 (s, 4H, Phthalimid-Protonen) |

Tabelle 1 (Fortsetzung)

| Verbindung | Ausbeute | Schmelzpunkt [°C] | $^1H$-NMR-Daten: δ (ppm) |
|---|---|---|---|
| 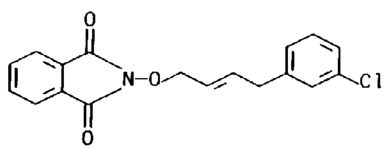 (7) | 98 | 95 - 97 | in DMSO-$d_6$: 3,38 (d, 2H, Ar-$CH_2$); 4,65 (d, 2H, -$CH_2$-O-); 5,7-6,1 (2m, 2H, -CH=CH-); 6,9-7,3 (m, 4H, Ar-H); 7,85 (s, 4H, Phthalimidprotonen) |

Tabelle 2

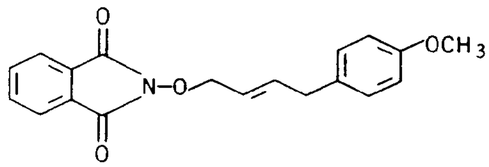

(8)

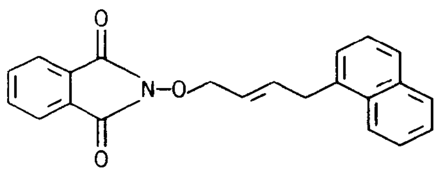

(9)

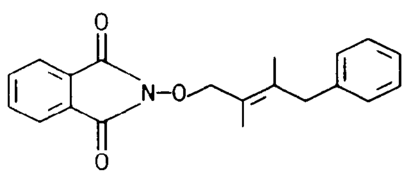

(10)

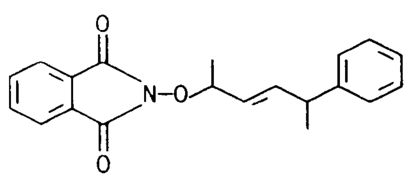

(11)

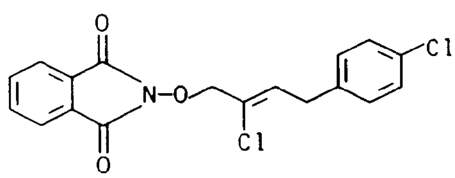

(12)

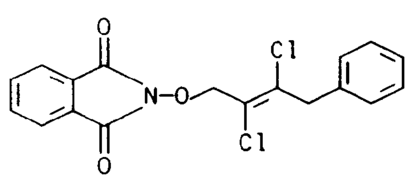

(13)

Beispiele 8 bis 11

Beispiel 11

E-N-(4-Phenyl-3-butenyloxy)-phthalimid (14)

Analog Beispiel 1 wurden 67 g (0,32 mol) E-4-Brom-1-phenyl-1-buten mit 29 g Kaliumcarbonat (0,21 mol)

und 51,9 g N-Hydroxyphthalimid (0,32 mol) in 320 ml N-Methylpyrrolidon umgesetzt. Das mittels Eiswasser ausgefällte Rohprodukt wurde in Dichlormethan aufgenommen und zweimal mit verdünnter Natronlauge gewaschen. Nach der üblichen Aufarbeitung wurde das Produkt in 56 % Ausbeute erhalten.

Auf analoge Weise wurden die Verbindungen (15), (16) und (17) erhalten (Strukturformeln, Ausbeuteangaben und physikalische Daten zu diesen Verbindungen s. Tabelle 3).

Auf analoge Weise können die in Tabelle 4 aufgeführten Verbindungen (18) bis (22) dargestellt werden.

Tabelle 3

| Verbindung | Ausbeute | Schmelzpunkt [°C] | 1H-NMR-Daten: δ (ppm) |
|---|---|---|---|
| 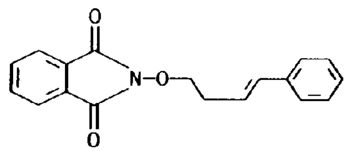 (14) | 56 % | 82 - 84 | 1H-NMR (in $CDCl_3$); δ (ppm):<br>2,7 (m, 2H, $=CH-CH_2$);<br>4,3 (t, 2H, $-CH_2-O-$);<br>6,2-6,4 (m, 1H, $=CH-CH_2$);<br>6,55 (d, 1H, Ar-CH=);<br>7,0-7,5 (m, 5H, Ar-H);<br>7,6-8,0 (m, 4H, Phthalimidprotonen) |
| 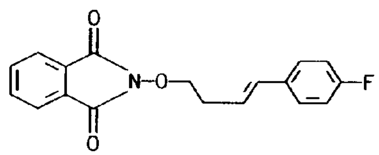 (15) | 28 % | 81 - 82 | in $CDCl_3$: 2,65 (m, 2H, $=CH-CH_2-$);<br>4,28 (t, 2H, $CH_2-O-$);<br>6,2-6,7 (2m, 2H, -CH=CH-);<br>7,15-7,24 (2m, 4H, Ar-H);<br>7,86 (s, 4H, Phthalimid-Protonen) |
| 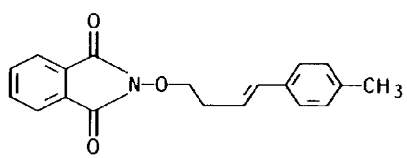 (16) | 21 % | 99 - 100 | in DMSO-$d_6$: 2,28 (s, 3H, $CH_3$);<br>2,62 (m, 2H, $=CH_2-CH_2$);<br>4,28 (t, 2H, $-CH_2-O-$);<br>6,2-6,35 (m, 1H, $=CH-CH_2$);<br>6,55 (d, 1H, Ar-CH=);<br>7,13+7,28 (2d, 4H, Ar-H);<br>7,85 (s, 4H, Phthalimid-Protonen) |

Tabelle 3 (Fortsetzung)

| Verbindung | Ausbeute | Schmelzpunkt [°C] | 1H-NMR-Daten: δ (ppm) |
|---|---|---|---|
| 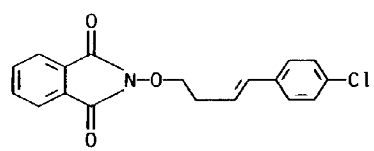 (17) | 29 % | 94 - 96 | in $CDCl_3$: 2,65 (m, 2H, $-CH=CH_2-$); 4,3 (t, 2H, $-CH_2-O-$); 6,3-6,7 (2m, 2H, $-CH=CH-$); 7,4 (m, 4H, Ar-H); 7,85 (s, 4H, Phthalimid-Protonen) |

Tabelle 4

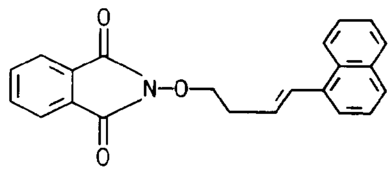

(18)

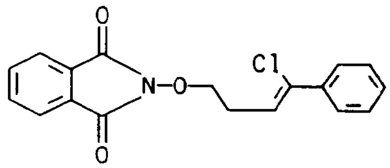

(19)

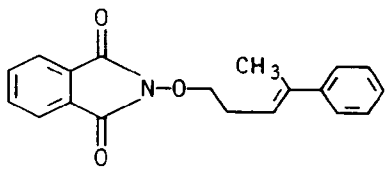

(20)

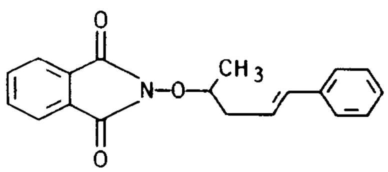

(21)

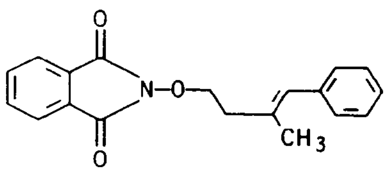

(22)

Beispiele 12 bis 19

Beispiel 12

E-4-Phenyl-2-butenyloxy-ammonium-oxalat (23)

55,5 g Verbindung (1) (0,19 mol) wurden in 190 ml Ethylacetat mit 11,6 g (0,19 mol) Ethanolamin versetzt und 5 h bei 60°C gerührt. Aus der abgekühlten Lösung wurde ausgefallenes N-(Hydroxyethyl)-phthalimid abfiltriert und zum Filtrat eine Lösung von 18,8 g Oxalsäure (0,21 mol) in 30 ml Ethylacetat gegeben. Das Produkt kristallisierte als Oxalat aus.

Auf analoge Weise wurden die Verbindungen (24), (25), (26), (27), (28), (29) und (30) hergestellt (Strukturformeln, Ausbeuten und die physikalischen Daten zu diesen Verbindungen können Tabelle 5 entnommen werden). Die Stöchiometrie der in Tabelle 5 aufgeführten Oxalate beruht auf den Ergebnissen von Elementaranalysen.

Auf analoge Weise können die Verbindungen (31) bis (34) aus Tabelle 6 hergestellt werden.

Beispiele 20 bis 22

Beispiel 20

E-4-Aminooxy-1-(4-fluorphenyl)-1-buten (35)

85,2 g (0,274 mol) der Verbindung (15) wurden zusammen mit 18,4 g (0,3 mol) Ethanolamin in 275 ml Ethylacetat 2 h auf 60°C erhitzt. Aus der abgekühlten Reaktionsmischung wurde ausgefallenes N-(Hydroxyethyl)-phthalimid abfiltriert. Das Filtrat wurde mit 200 ml Dichlormethan versetzt und viermal mit Wasser gewaschen. Nach der üblichen Aufarbeitung wurde das Produkt in quantitativer Ausbeute als Öl isoliert.

Auf analoge Weise wurden die Verbindungen 36 und 37 hergestellt (Strukturformeln, Ausbeuten und NMR-Daten sind in Tabelle 7 aufgeführt).

Die Verbindungen (38) bis (42) der Tabelle 8 können analog hergestellt werden.

Tabelle 5

| Verbindung | Ausbeute | Schmelzpunkt [°C] | 1H-NMR-Daten: δ (ppm) |
| --- | --- | --- | --- |
| 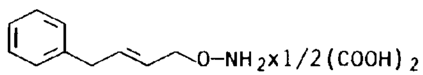 (23) | 95 % | 127 - 129 | 1H-NMR (in DMSO-$d_6$): δ (ppm): 3,25 (d, 2H, Ar-$CH_2$-); 4,18 (d, 2H, $CH_2$-O-); 5,5-5,7 (m, 1H, Ar-$CH_2$-CH=); 5,7-6,0 (m, 1H, =CH-$CH_2$-O); 7,0-7,4 (m, 5H, Ar-H); 9,0 (breites s, 3H, $NH_3^+$) |
| 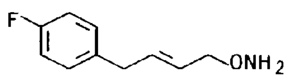 (24) | 85 % | 60 - 62 | in DMSO-$d_6$: 3,35 (d, 2H, Ar-$CH_2$); 4,2 (d, 2H, $CH_2$-O-); 5,5-5,7 (m, 1H, Ar-$CH_2$-CH=); 5,7-6,0 (m, 1H, =CH-$CH_2$-O); 7,0-7,3 (m, 4H, Ar-H); 9,05 (s, 3H, $NH_3^+$) |
| 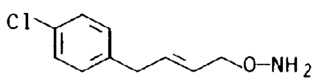 (25) | 90 % | 114 - 116 | in DMSO-$d_6$: 3,4 (d, 2H, Ar-$CH_2$); 4,25 (d, 2H, $CH_2$-O); 5,5-6,0 (2m, 2H, -CH=CH-); 7,2-7,4 (2d, 4H, Ar-H); 10,0 (breites s, 4H, $NH_3^+$+COOH) |

Tabelle 5 (Fortsetzung)

| Verbindung | Ausbeute | Schmelzpunkt [°C] | $^1$H-NMR-Daten: δ (ppm) |
|---|---|---|---|
| 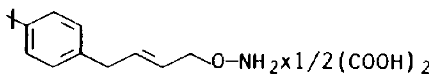 (26) | 80 % | 143 - 146 | in DMSO-$d_6$: 1,12 (s, 9H, tert.-Butyl); 3,35 (d, 2H, Ar-$CH_2$); 4,15 (d, 2H, $CH_2$-O-); 5,5-6,0 (2m, 2H, CH=CH-); 7,0-7,4 (2d, 4H, Ar-H); 7,4-8,0 (breites s, 3H, $NH_3^+$) |
| 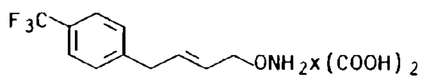 (27) | 91 % | 120 - 122 | in DMSO-$d_6$: 3,5 (d, 2H, Ar-$CH_2$); 4,22 (d, 2H, $CH_2$-O); 5,5-6,1 (2m, 2H, Ar-CH=CH-); 7,0-7,4 (2d, 4H, Ar-H); 7,7-8,0 (breites s, 3H, $NH_3^+$) |
| 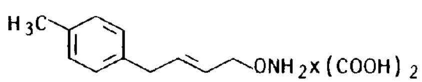 (28) | 96 % | 115 - 118 | in DMSO-$d_6$: 2,25 (s, 3H, Ar-$CH_3$); 3,32 (d, 2H, Ar-$CH_2$); 4,22 (d, $CH_2$-O-); 5,5-6,0 (2m, 2H, -CH=CH-); 7,1 (s, 4H, Ar-H); 8,8 (breites S; $NH_3^+$, COOH) |

Tabelle 5 (Fortsetzung)

| Verbindung | Ausbeute | Schmelzpunkt [°C] | 1H-NMR-Daten: δ (ppm) |
|---|---|---|---|
| 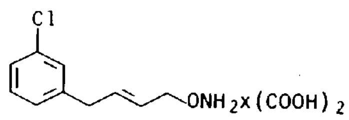 (29) | 67 % | 99 - 104 | in DMSO-$d_6$: 3,4 (d, 2H, Ar-$CH_2$); 4,25 (d, 2H, $CH_2$-O); 5,5-6,1 (2m, 2H, -CH=CH-); 7,0-7,5 (m, 4H, Ar-H); 8,1 (breites s, $NH_3^+$, COOH) |
| 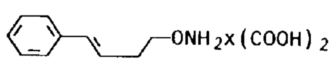 (30) | 78 % | 112 - 114 | in DMSO-$d_6$: 2,3-2,6 (m, 2H-$CH_2$-CH=); 3,8 (t, 2H, $CH_2$-O-); 6,1-6,4 (m, 1H, =CH-$CH_2$-); 6,5 (d, 1H, Ar-CH=); 7,0-7,5 (m, 5H, Ar-H); 7,8 (breites s, $NH_3^+$+COOH) |

Tabelle 6

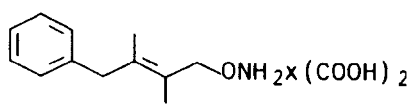

(31)

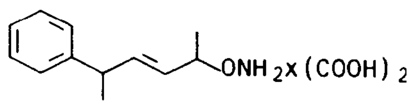

(32)

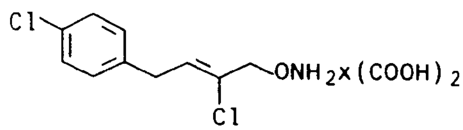

(33)

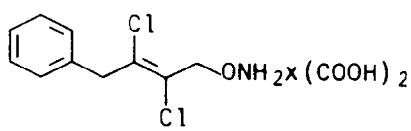

(34)

Tabelle 7

| Verbindung | Ausbeute | Schmelzpunkt [°C] | 1H-NMR-Daten: δ (ppm) |
|---|---|---|---|
| 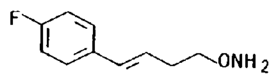 (35) | 98 % | Öl | 1H-NMR (in DMSO-$d_6$): δ (ppm):<br>2,5 (m, 2H, $=CH-CH_2-$);<br>3,28 (t, 2H, $CH_2-O$);<br>5,45 (breites s, 2H, $-ONH_2$);<br>6,0-6,2 (m, 1H, $=CH-CH_2$);<br>6,22 (d, 1H, Ar-CH=);<br>6,9-7,4 (2m, 4H, Ar-H). |
| 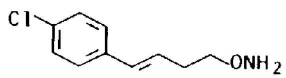 (36) | 92 % | Öl | in $CDCl_3$: 2,5 (q, 2H, $-CH_2-CH=$);<br>3,78 (t, 2H, $-CH_2-O-$);<br>5,4 (breites s, 2H, $NH_2$);<br>6,1-6,5 (m, 2H, -CH=CH-);<br>7,1-7,3 (m, .4H, Ar-H) |
| 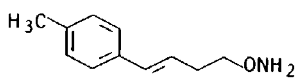 (37) | 98 % | Öl | in $CDCl_3$: 2,35 (s, 3H, $CH_3$);<br>2,55 (q, 2H, $-CH_2-CH=$);<br>3,83 (t, 2H, $-CH_2-O-$);<br>5,45 (breites s, 2H, $NH_2$);<br>6,0-6,6 (m, 2H, -CH=CH-);<br>7,1-7,4 (2d, 4H, Ar-H) |

Tabelle 8

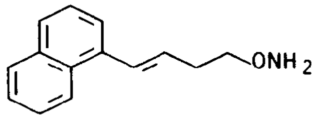

(38)

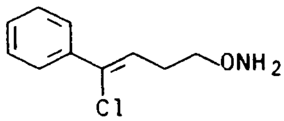

(39)

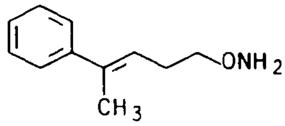

(40)

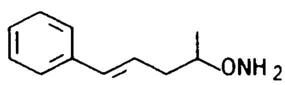

(41)

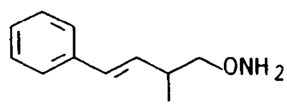

(42)

## Patentansprüche

1. Hydroxylaminderivate der allgemeinen Formel I

Aryl-A-O-B I

in der

Aryl einen ein- oder zweikernigen isocyclischen aromatischen Rest bedeutet, wobei dieser Rest folgende Substituenten tragen kann

- bis zu 5 Halogenatome und/oder
- bis zu 3 der nachstehenden Gruppen:

Nitro, Nitril, Hydroxy, Amino, $C_1$- bis $C_7$-Monoalkylamino, $C_2$- bis $C_{14}$-Dialkylamino, $C_1$- bis $C_7$-Alkyl, $C_1$- bis $C_7$-Alkoxy, $C_1$- bis $C_7$-Halogenalkoxy, $C_1$- bis $C_7$-Halogenalkyl, $C_1$- bis $C_7$-Alkylsulfido, $C_1$- bis $C_7$-Halogenalkylsulfido, $C_1$- bis $C_7$-Acyl, $C_1$- bis $C_7$-Alkoxycarbonyl, Carboxyl, $C_3$- bis $C_6$-Cycloalkyl- und/oder Phenyl, in der

A eine 1,4-Butenylengruppe der allgemeinen Formeln IIa oder IIb ist,

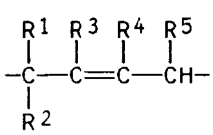

IIa

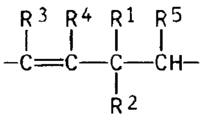

IIb

in der die Reste $R^1$ bis $R^4$ für Wasserstoff, Halogen, $C_1$-$C_6$-Alkyl und/oder $C_1$-$C_6$-Alkenyl stehen und $R^5$ Wasserstoff oder $C_1$-$C_6$-Alkyl bedeutet
und in der
B entweder für die $NH_2$-Gruppe oder für eine Dicarbonsäureimidgruppe der allgemeinen Formel III steht,

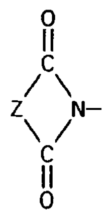

III

in der Z für einen Phenylen-, Naphthylen, Pyridinylen-, Cyclopentylen-, Cyclohexylen-, Cyclohexenylen-, $C_2$- bis $C_4$-Alkenylen- oder $C_1$- bis $C_4$-Alkylenrest steht, wobei diese Reste Z unsubstituiert sind oder 1, 2, 3 oder 4 Halogen-, $C_1$- bis $C_4$-Alkyl- und/oder $C_1$- bis $C_4$-Halogenalkyl-Substituenten tragen können,
sowie, falls B eine Aminogruppe bedeutet, Salze der Verbindungen I.

2. Substituierte Hydroxylaminderivate nach Anspruch 1, in denen
Aryl eine Phenyl- oder Naphthylgruppe ist oder für eine 1, 2 oder 3 Substituenten tragende Phenyl- oder Naphthylgruppe steht, deren Substituenten Fluor, Chlor, Brom, $C_1$- bis $C_4$-Monoalkylamino, $C_2$- bis $C_8$-Dialkylamino, $C_1$- bis $C_4$-Alkyl, $C_1$- bis $C_4$-Alkoxy, $C_1$- bis $C_4$-Alkylsulfido, $C_1$- bis $C_4$-Halogenalkyl, $C_1$- bis $C_4$-Halogenalkoxy, $C_1$- bis $C_4$- Halogenalkylsulfido, $C_1$- bis $C_4$-Acyl, Carboxyl- und/oder $C_1$- bis $C_4$-Alkoxycarbonyl sind,
A eine 1,4-Butenylengruppe der allgemeinen Formeln IIa oder IIb ist, worin
- $R^1$, $R^2$ und $R^5$ Wasserstoff und/oder $C_1$- bis $C_4$-Alkyl bedeuten
- $R^3$ und $R^4$ Wasserstoff, Halogen und/oder $C_1$- bis $C_4$-Alkyl- sind

und
B eine Aminogruppe ist.

3. Verfahren zur Herstellung der substituierten Hydroxylaminderivate der allgemeinen Formel I, dadurch gekennzeichnet, daß man Verbindungen der allgemeinen Formel IV

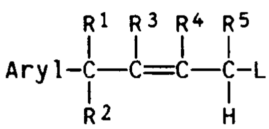

IV

oder V,

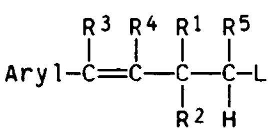

V

in denen L eine unter nucleophilen Bedingungen substituierbare Abgangsgruppe ist, zunächst mit einem Hydroxyimid der allgemeinen Formel VI

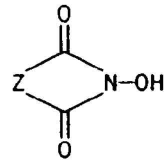

VI

in Gegenwart eines Lösungsmittels und einer Base bei Temperaturen von 0 bis 140°C umsetzt und aus den erhaltenen Imidethern die Hydroxylaminderivate I, in denen B für die $NH_2$-Gruppe steht, mittels Basen oder Säuren freisetzt.

4. Verfahren zur Herstellung der substituierten Hydroxylaminderivate der allgemeinen Formel Ia,

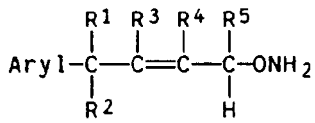

Ia

dadurch gekennzeichnet, daß man eine Verbindung der allgemeinen Formel VII,

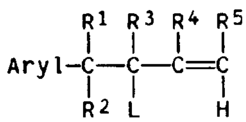

VII

worin L eine unter nucleophilen Bedingungen substituierbare Abgangsgruppe ist oder Mischungen der Verbindung der allgemeinen Formel VII mit der isomeren Verbindung der allgemeinen Formel IV

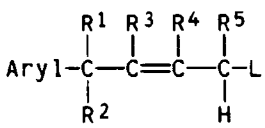

IV

zunächst mit einem Hydroxyimid der allgemeinen Formel VI

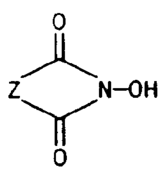

VI

in Gegenwart eines Lösungsmittels und einer Base bei Temperaturen von 0 bis 140°C umsetzt und aus den erhaltenen Imidethern die Hydroxylaminderivate Ia mittels Basen oder Säuren freisetzt.

## Claims

1. A hydroxylamine derivative of the general formula I

Aryl-A-O-B    I

where

Aryl is a mononuclear or dinuclear isocyclic aromatic radical which may be substituted by the following substituents:

- up to 5 halogen atoms and/or
- up to 3 of the following groups:

nitro, nitrile, hydroxyl, amino, $C_1$-$C_7$,-monoalkylamino, $C_2$-$C_{14}$-dialkylamino, $C_1$-$C_7$-alkyl, $C_1$-$C_7$-alkoxy, $C_1$-

$C_7$-haloalkoxy, $C_1$-$C_7$-haloalkyl, $C_1$-$C_7$-alkylsulfido, $C_1$-$C_7$-haloalkylsulfido, $C_1$-$C_7$-acyl, $C_1$-$C_7$-alkoxycarbonyl, carboxyl, $C_3$-$C_6$-cycloalkyl and/or phenyl,

A is a 1,4-butenylene group of the general formula IIa or IIb

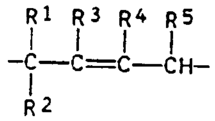

IIa

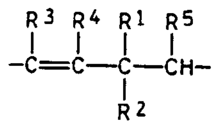

IIb

where each of $R^1$ to $R^4$ is hydrogen, halogen, $C_1$-$C_6$-alkyl or $C_1$-$C_6$-alkenyl and $R^5$ is hydrogen or $C_1$-$C_6$-alkyl, and

B is either an $NH_2$ group or a dicarboximide group of the general formula III

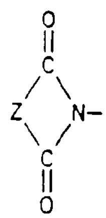

III

where Z is phenylene, naphthylene, pyridinylene, cyclopentylene, cyclohexylene, cyclohexenylene, $C_2$-$C_4$-alkenylene or $C_1$-$C_4$-alkylene, which may each be unsubstituted or substituted by 1, 2, 3 or 4 halogen, $C_1$-$C_4$-alkyl and/or $C_1$-$C_4$-haloalkyl substituents,

and, if B is amino, or a salt thereof.

2. A substituted hydroxylamine derivative as claimed in claim 1, where

Aryl is phenyl or naphthyl or monosubstituted, disubstituted or trisubstituted phenyl or naphthyl, the substituents being fluorine, chlorine, bromine, $C_1$-$C_4$-monoalkylamino, $C_2$-$C_8$-dialkylamino, $C_1$-$C_4$-alkyl, $C_1$-$C_4$-alkoxy, $C_1$-$C_4$-alkylsulfido, $C_1$-$C_4$-haloalkyl, $C_1$-$C_4$-haloalkoxy, $C_1$-$C_4$-haloalkylsulfido, $C_1$-$C_4$-acyl, carboxyl and/or $C_1$-$C_4$-alkoxycarbonyl,

A is a 1,4-butenylene group of the general formulae IIa or IIb where

– $R^1$, $R^2$ and $R^5$ are each hydrogen or $C_1$-$C_4$-alkyl and

– $R^3$ and $R^4$ are each hydrogen, halogen or $C_1$-$C_4$-alkyl,

and

B is amino.

3. A process for preparing a substituted hydroxylamine derivative of the general formula I, which comprises reacting a compound of the general formula IV or

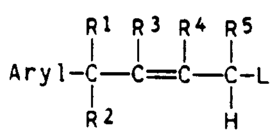

IV

or

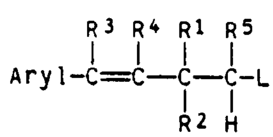

V

where L is a nucleophilically displaceable leaving group, with a hydroxyimide of the general formula VI

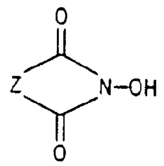

VI

in the presence of a solvent and a base at from 0 to 140°C and treating the resulting imide ether with a base or an acid to liberate the hydroxylamine derivative I where B is $NH_2$.

4. A process for preparing a substituted hydroxylamine derivative of the general formula Ia

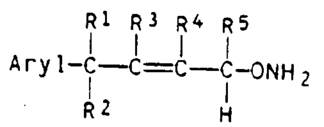

Ia

which comprises reacting a compound of the general formula VII

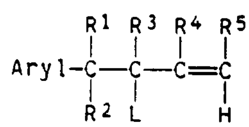

VII

where L is a nucleophilically displaceable leaving group or a mixture of a compound of the general formula VII with the isomeric compound of the general formula IV

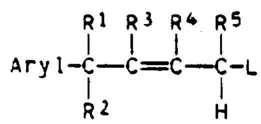

IV

with a hydroxyimide of the general formula VI

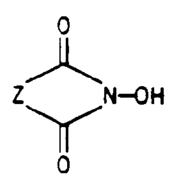

VI

in the presence of a solvent and a base at from 0 to 140°C and treating the resulting imide ether with a base or an acid to liberate the hydroxylamine derivative Ia.

## Revendications

1. Dérivés d'hydroxylamine de formule générale I

Aryle-A-O-B I,

dans laquelle

aryle représente un reste aromatique isocyclique à un ou deux noyaux, ce reste pouvant porter les substituants suivants :

– jusqu'à 5 atomes d'halogène et/ou

– jusqu'à trois des groupes indiqués ci-après :

nitro, nitrile, hydroxy, amino, monoalkylamino en C1 à C7, dialkylamino en C2 à C14, alkyle en C1 à C7, alcoxy en C1 à C7, halogènalcoxy en C1 à C7, halogènalkyle en C1 à C7, alkysulfido en C1 à C7, halogènalkylsulfido en C1 à C7, acyle en C1 à C7, alcoxycarbonyle en C1 à C7, carboxyle, cycloylkyle en C3

à C6 et/ou phényle, dans laquelle
A est un groupe 1,4-buténylène de formule générale IIa ou IIb

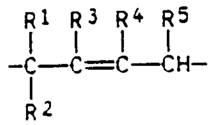

IIa

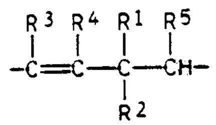

IIb

où les restes $R^1$ à $R^4$ sont mis pour hydrogène, halogène, alkyle en C 1-C 6 et/ou alcényle en C 1-C 6 et $R^5$ représente hydrogène ou alkyle en C 1-C 6,
et où
B est mis pour le groupe $NH_2$ ou pour un groupe imide d'acide dicarboxylique de formule générale III

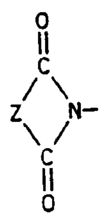

III

dans laquelle Z est mis pour un reste phénylène, naphtylène, pyridinylène, cyclopentylène, cyclohexylène, cyclohexénylène, alcénylène en C2 à C4 ou alkylène en Cl à C4 ces restes Z étant non substitués ou pouvant porter 1, 2, 3 ou 4 substituants halogène, alkyle en C1 à C4 et/ou halogènalkyle en C1 à C4,
et, au cas où B représente un groupe amino, les sels des composés I.

2. Dérivés d'hydroxylamine substitués selon la revendication 1, dans lesquels
aryle est un groupe phényle ou naphtyle ou est mis pour un groupe phényle ou naphtyle portant 1, 2 ou 3 substituants, les substituants étant fluor, chlore, brome, monoalkylamino en C1 à C4, dialkylamino en C2 à C8, alkyle en C1 à C4, alcoxy en C1 à C4, alkylsulfido en C1 à C4, halogènalkyle en C1 à C4, halogènalcoxy en C1 à C4, halogènalkylsulfido en C1 à C4, acyle en C1 à C4, carboxyle et/ou alcoxycarbonyle en C1 à C4,
A est un groupe 1,4-buténylène de formule générale IIa ou IIb où

- $R^1$, $R^2$ et $R^5$ représentent hydrogène et/ou alkyle en C1 à C4
- $R^3$ et $R^4$ sont mis pour hydrogène, halogène et/ou alkyle en C1 à C4

et
B est un groupe amino.

3. Procédé de préparation des dérivés d'hydroxylamine substitués de formule générale I, caractérisé par le fait que l'on fait d'abord réagir, en présence d'un solvant et d'une base, à des températures de 0 à 140 degrés C, des composés des formule générale IV

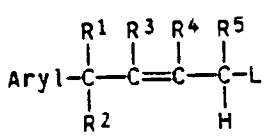

IV

ou

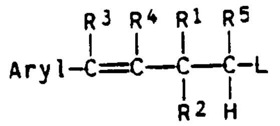

V

dans lesquelles L est un groupe substituable dans des conditions nucléophiles, avec un hydroxyimide de formule générale VI

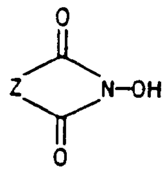

VI

et à partir des imido-éthers ainsi obtenus, on met en liberté, au moyen de bases ou d'acides, les dérivés d'hydroxylamine I, dans lesquels B est mis pour le groupe $NH_2$.

4. Procédé de préparation des dérivés d'hydroxylamine substitués, de formule générale Ia

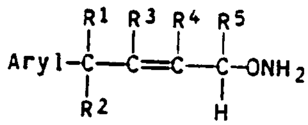

Ia

caractérisé par le fait que l'on fait d'abord réagir, en présence d'un solvant et d'une base, à des températures de 0 à 140 degrés C, un composé de formule générale VII

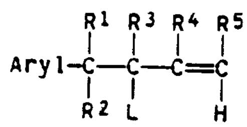

VII

dans laquelle L est un groupe substituable dans des conditions nucléophiles, ou des mélanges du composé de formule générale VII avec le composé isomère de formule générale IV

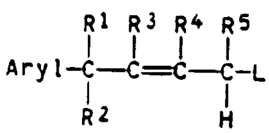

IV

avec un hydroxyimide de formule générale VI

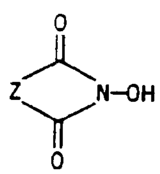

VI

et à partir des imido-éthers ainsi obtenus, on met les dérivés d'hydroxylamine Ia en liberté au moyen de bases ou d'acides.